# EUROPEAN PATENT APPLICATION

(11) **EP 4 470 483 A1**
(43) Date of publication of application: **04.12.2024**
(21) Application number: 22923775.5
(22) Date of filing: 26.01.2022
(51) Int. Cl.: A61B 17/17

(54) **GUIDE DEVICE**

(71) Applicant: Olympus Terumo Biomaterials Corp., Tokyo 151-0073 (JP); National University Corporation Tokyo Medical and Dental University, Tokyo 113-8510 (JP)
(72) Inventor: FUKUHARA, Tomohiko, Tokyo 151-0073 (JP); IWANAGA, Toshihisa, Tokyo 151-0073 (JP); KOGA, Hideyuki, Tokyo 113-8510 (JP)
(74) Representative: Nederlandsch Octrooibureau
(86) International application number: PCT/JP2022/002795
(87) International publication number: WO 2023/144907

(57) **Abstract**

Provided is a guide device (1) that guides a drill (14) that drills a second bone hole in a bone in which a first bone hole is formed. The guide device (1) includes: an elongated protective portion (2) that is inserted into the first bone hole and that defines, in an interior of the first bone hole, a pathway that extends in a longitudinal direction of the first bone hole and through which a suture passes; a guide portion (3) that has a guide hole that specifies a guide axis (G) and that guides the drill (14) passing through the guide hole along the guide axis (G); and an arm portion (4) that connects the protective portion (2) with the guide portion (3). The guide axis (G) is disposed at a twist position with respect to the protective portion (2), and the drill (14) guided along the guide axis (G) passes through a position at which the drill does not interfere with the suture that passes through the pathway.

## Description

### {Technical Field}

The present invention relates to a guide device and, in particular, relates to a guide device that guides a bone drill.

### {Background Art}

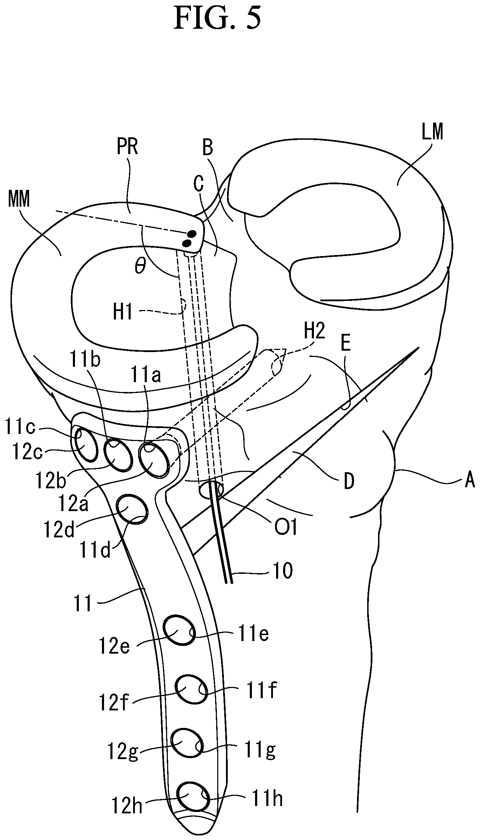

In the related art, a pull-out method is generally employed as a restoration technique for a medial meniscus posterior root tear (MMPRT). As shown in Fig. 5, in a pull-out method, a torn end of a posterior root (PR) of a medial meniscus (MM) is pulled forward by means of a suture 10, the torn end is reset and fused to a posterior root attachment portion C of a joint surface B. In the pull-out method, a bone hole H1 is fabricated from a front surface of a tibia A to the posterior root attachment portion C, the suture 10 bridged over the torn end is pulled out forward with respect to the tibia A via the bone hole H1, and the suture 10 is secured to the front surface of the tibia A.

Meanwhile, there is a known guide device that guides a drill for drilling a hole in a bone (for example, see Patent Literature 1). The guide device of Patent Literature 1 is used when anterior cruciate ligament reconstruction and a high tibial osteotomy (HTO) are simultaneously performed, and a plurality of drills are guided so that a bone hole for the anterior cruciate ligament reconstruction and a bone hole for the high tibial osteotomy do not interfere with each other.

### {Citation List}

### {Patent Literature}

{PTL 1} Japanese Unexamined Patent Application, Publication No. 2012-165977

### {Summary of Invention}

### {Technical Problem}

As shown in Fig. 5, in the case in which knee osteoarthritis is involved in addition to the MMPRT, there are cases in which the pull-out method and an HTO are simultaneously performed. In an HTO, an osteotomy is applied to the tibia A from an inside thereof, an osteotomy portion D is opened up, and thus, the tibia A is corrected. In order to immobilize the corrected tibia A, a bone plate 11 disposed on an inside surface of the tibia A is secured to the tibia A by means of screws 12a, 12b, 12c, ···, and 12h on a proximal side and a distal side of the osteotomy portion D.

In this case, the screw 12a on the proximal side and the front side is often disposed so as to pass through the vicinity of the suture 10. Therefore, a surgeon must accurately determine the position of a bone hole H2 for the screw 12a with respect to the bone hole H1 through which the suture 10 passes and carefully drill the bone hole H2 so that the drill does not interfere with the suture 10. Such work imposes a heavy burden on the surgeon.

The present invention has been conceived in light of the above-described circumstances, and an object thereof is to provide a guide device with which it is possible to easily provide a second bone hole in the vicinity of a first bone hole through which a suture passes, while preventing interference between a drill and the suture.

### {Solution to Problem}

An aspect of the present invention is a guide device that guides a drill that drills a second bone hole in a bone in which a first bone hole is formed, the guide device including: an elongated protective portion that is inserted into the first bone hole and that defines, in an interior of the first bone hole, a pathway that extends in a longitudinal direction of the first bone hole and through which a suture passes; a guide portion that has a guide hole that specifies a guide axis and that guides the drill passing through the guide hole along the guide axis; and an arm portion that connects the protective portion with the guide portion, wherein the guide axis is disposed at a twist position with respect to the protective portion, and the drill guided along the guide axis passes through a position at which the drill does not interfere with the protective portion and the pathway.

### {Advantageous Effects of Invention}

The present invention affords an effect in that it is possible to easily provide a second bone hole in the vicinity of a first bone hole through which a suture passes, while preventing interference between a drill and the suture.

### {Brief Description of Drawings}

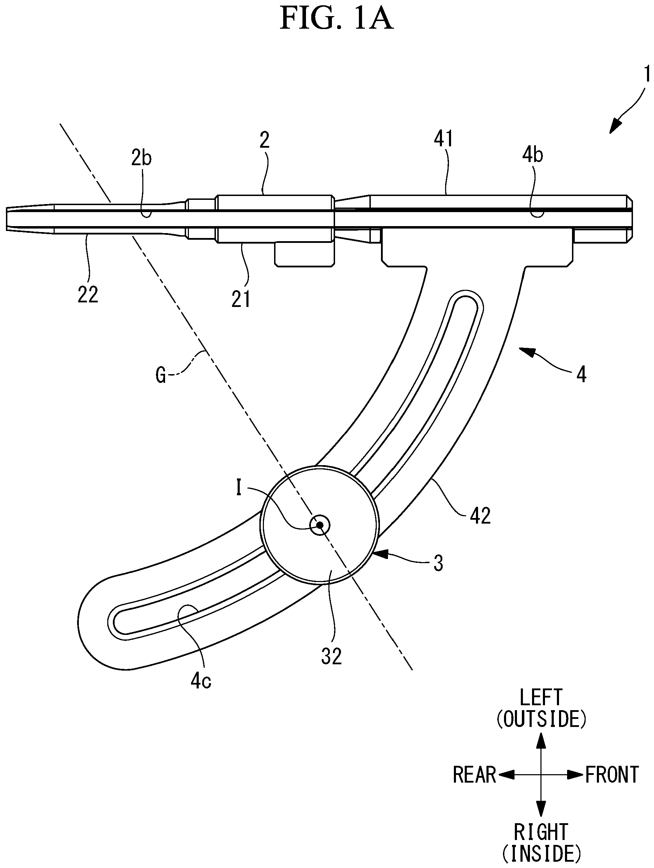

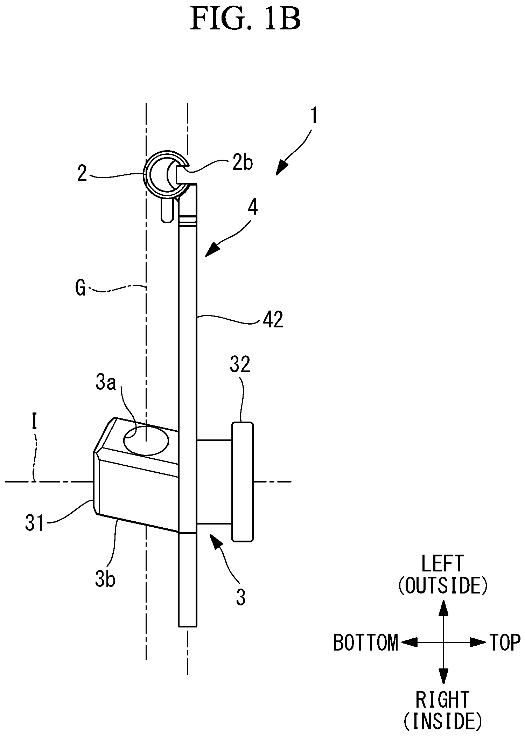

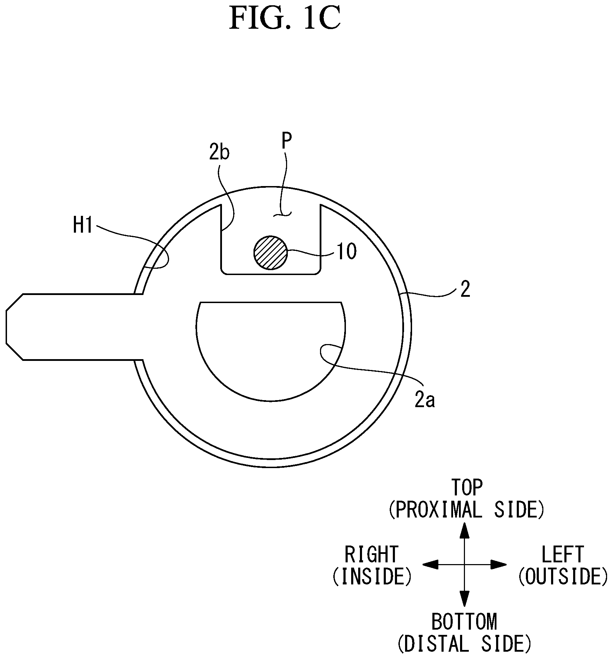

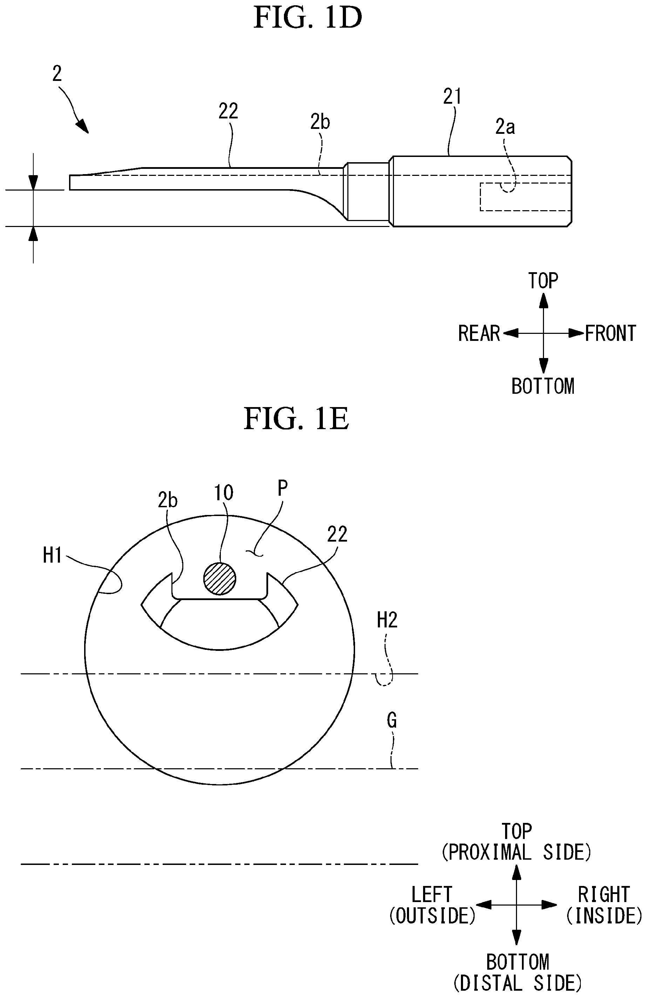

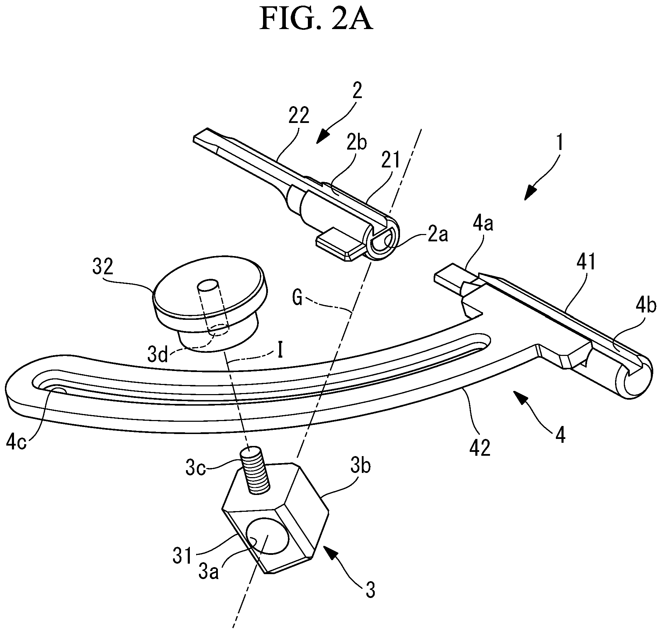

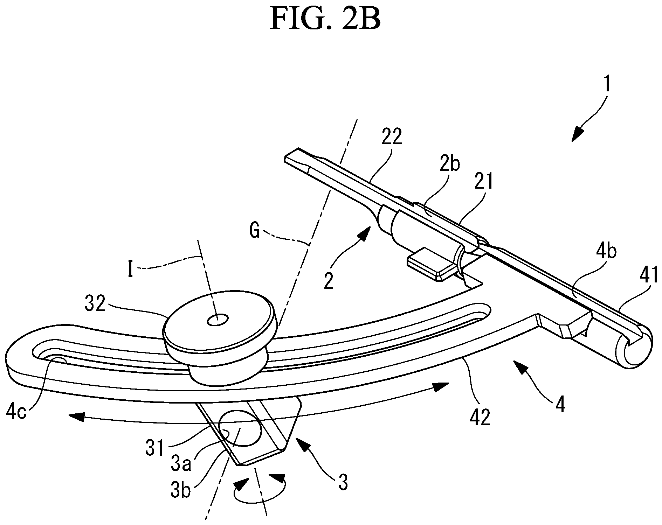

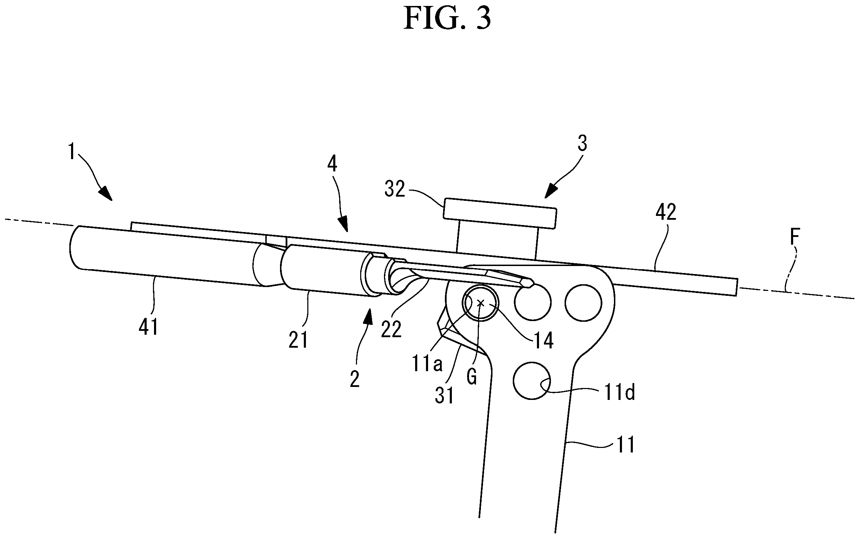

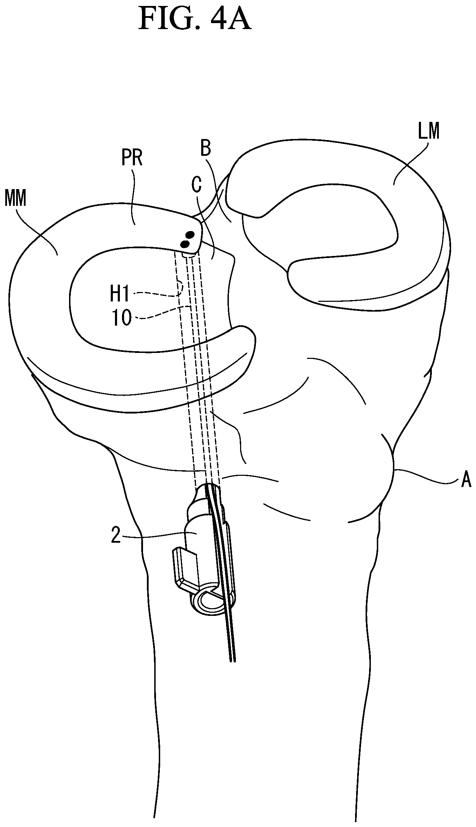

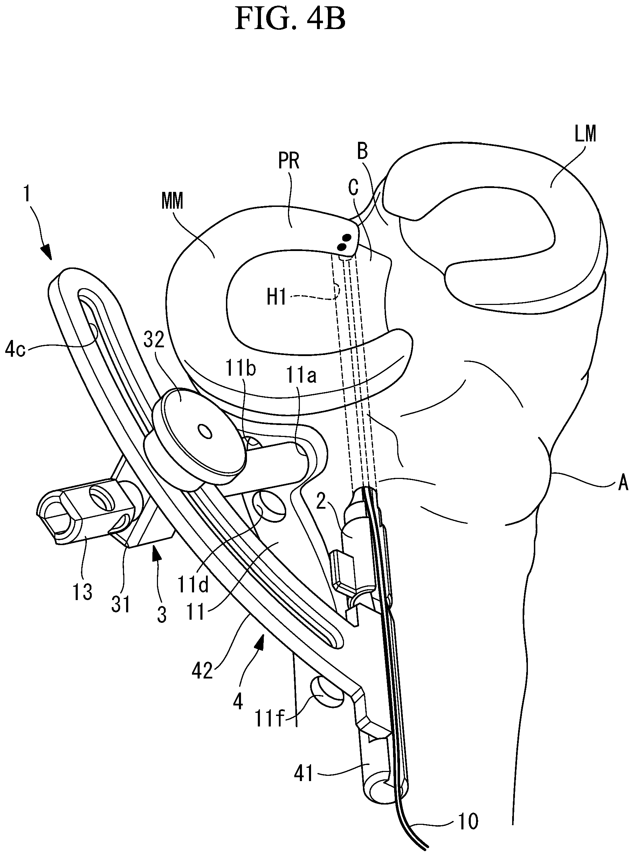

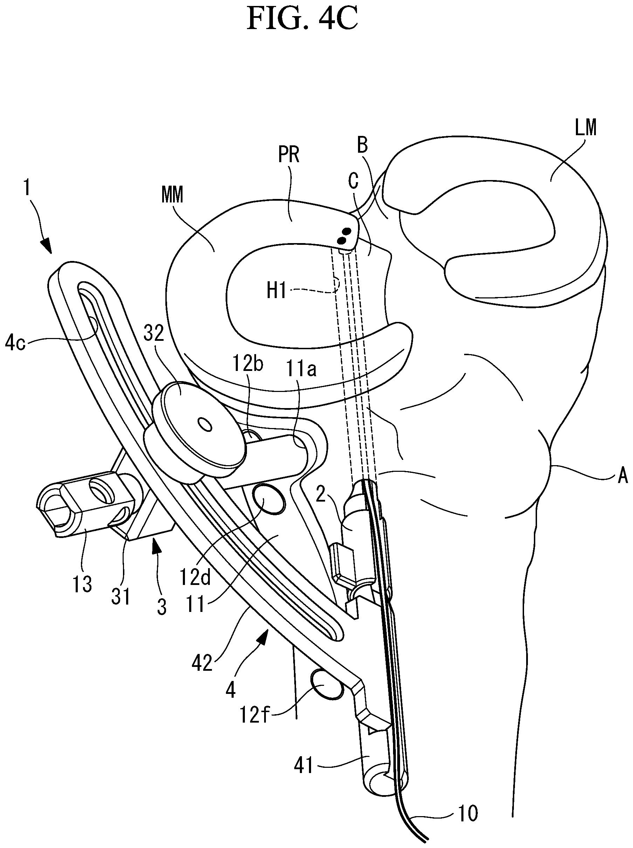

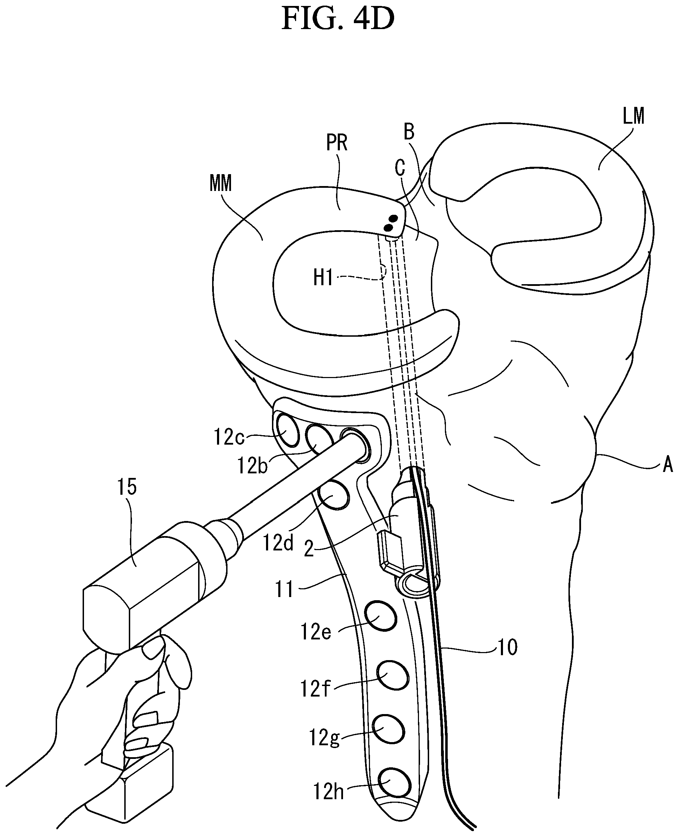

{Fig. 1A} Fig. 1A is a plan view of a guide device according to an embodiment of the present invention.
{Fig. 1B} Fig. 1B is a back view of the guide device in Fig. 1A.
{Fig. 1C} Fig. 1C is a diagram in which a protective portion inserted into a bone hole for a pull-out method is viewed from a front side.
{Fig. 1D} Fig. 1D is a side view of the protective portion of the guide device in Fig. 1A.
{Fig. 1E} Fig. 1E is a diagram in which the protective portion inserted into the bone hole for a pull-out method is viewed from a rear side.
{Fig. 2A} Fig. 2A is an exploded perspective view of the guide device in Fig. 1A.
{Fig. 2B} Fig. 2B is an assembly perspective view of the guide device in Fig. 1A.
{Fig. 3} Fig. 3 is a side view of the guide device viewed along a guide axis.
{Fig. 4A} Fig. 4A is a diagram for explaining a method for using the guide device.
{Fig. 4B} Fig. 4B is a diagram for explaining the method for using the guide device.
{Fig. 4C} Fig. 4C is a diagram for explaining the method for using the guide device.
{Fig. 4D} Fig. 4D is a diagram for explaining the method for using the guide device.
{Fig. 5} Fig. 5 is a diagram for explaining a pull-out method and an HTO.

### {Description of Embodiment}

A guide device 1 according to an embodiment of the present invention will be described below with reference to the drawings.

As shown in Fig. 5, the guide device 1 according to this embodiment is used in a surgery in which a pull-out method and a high tibial osteotomy (HTO) are simultaneously performed. A first bone hole H1 for the pull-out method and a second bone hole H2 for the HTO both are formed in a proximal bone end portion of a tibia A. The guide device 1 is for guiding a drill that drills the bone hole H2 in the proximal bone end portion in which the bone hole H1 is formed.

The pull-out method and the HTO will be described before describing the guide device 1.

The pull-out method is one of restoration techniques for a medial meniscus posterior root tear (MMPRT). In the pull-out method, the bone hole H1 is formed from a front surface of the tibia A to a posterior root attachment portion C of a joint surface B, a suture 10 bridged over a torn end of a posterior root (PR) of a medial meniscus (MM) is pulled out to a front side of the tibia A via the bone hole H1, and the suture 10 is secured to the front surface of the tibia A in the state in which the suture 10 is pulled in an anteromedial direction of the tibia A. In Fig. 5, LM indicates a lateral meniscus, and the left side, the right side, the front side, and the back side of the figure correspond to the inside, the outside, the front side, and the rear side of the tibia A, respectively.

In the HTO, a bone plate 11, bone screws 12a-12h (hereinafter, these screws will also be collectively referred to as the bone screws 12) and a drill sleeve 13 (see Figs. 4B and 4C) are used.

The bone plate 11 is a substantially T-shaped plate-like member and has a plurality of screw holes 11a-11h that pass through the bone plate 11 in a thickness direction thereof. The screw holes 11a-11h are screw holes to which male screws (not shown) provided in head portions of the screws 12 and a distal-end portion of the drill sleeve 13, respectively, are connected.

The drill sleeve 13 is a cylindrical member that guides a drill. By connecting the distal-end portion of the drill sleeve 13 to any one of the screw holes 11a-11h, the drill sleeve 13 is secured to the bone plate 11 so that the drill sleeve 13 and one of the screw holes 11a-11h are coaxially aligned.

In the HTO, an osteotomy is applied to the tibia A from an inside thereof and an artificial bone or an autologous bone is inserted into an opened wedge-shaped osteotomy portion D. Next, the bone plate 11 is temporarily secured, as needed, to the inside surface of the tibia A by means of a wire or the like, and the plurality of bone holes H2 are formed one at a time in the tibia A by means of a drill via the interior of the drill sleeve 13 secured to any one of the screw holes 11a-11h. Next, the bone plate 11 is secured to the tibia A by screwing the screws 12 into the bone holes H2 until the head portions are connected to the screw holes 11a-11h.

In order to firmly secure the bone plate 11 to the proximal bone end portion of the tibia A, the screw 12d adjacent to a proximal-side osteotomy surface E is preferably disposed so as not to interfere with the osteotomy surface E, and, for this reason, the screw 12a on the most proximal side and the most front side preferably passes through the vicinity of the bone hole H1 for the pull-out method.

The guide device 1 of this embodiment is used, in particular, to form the bone hole H2 for the screw 12a and to fasten the screw 12a into the bone hole H2, while preventing the drill and the screw 12a from interfering with the suture 10.

Next, the guide device 1 of this embodiment will be described.

As shown in Figs. 1A to 2B, the guide device 1 includes: a protective portion 2 for protecting the suture 10 in the bone hole H1 from the drill and the screws 12; a guide portion 3 that guides the drill along a guide axis G; and an arm portion 4 that connects the protective portion 2 with the guide portion 3.

The protective portion 2, the guide portion 3, and the arm portion 4 are formed from separate members, and the protective portion 2 and the guide portion 3 are attached to the arm portion 4 in a detachable manner. Figs. 1A, 1B, and 2B show the guide device 1 in an integrally assembled state and Fig. 2A shows a state in which the guide device 1 is in a disassembled state.

In the guide device 1, there are a top-to-bottom direction, a left-to-right direction, and a front-to-rear direction that are orthogonal to each other. The top-to-bottom direction, the left-to-right direction, and the front-to-rear direction of the guide device 1 substantially correspond to the longitudinal direction, the left-to-right direction, and the front-to-rear direction of the tibia A, respectively. Specifically, the top side, the bottom side, the left side, the right side, the front side, and the rear side of the guide device 1 substantially correspond to the proximal side, the distal side, the left side, the right side, the front side, and the rear side of the tibia A, respectively. Fig. 1A is a plan view of the guide device 1 viewed from the top side and Fig. 1B is a back view of the guide device 1 viewed from the rear side.

The guide device 1 shown in Figs. 1A-2B is for the tibia A of the left leg, and the left side and the right side of the guide device 1 correspond to an outside and an inside of the tibia A of the left leg, respectively. In the description below, the left side and the right side of the guide device 1 will also be referred to as the outside and the inside, respectively. A guide device 1 for the tibia A of the right leg has a structure in which the left and the right of the guide device 1 in Figs. 1A-2B are inverted.

The protective portion 2 is an elongated member that extends in the front-to-rear direction and that is inserted into the bone hole H1 for the pull-out method. A basal-end portion (front-end portion) of the protective portion 2 is provided with a connecting portion 2a (see Fig. 2A) to be connected to the arm portion 4.

As shown in Fig. 1C, the protective portion 2 defines, in the bone hole H1, a pathway P that extends in the longitudinal direction of the bone hole H1 and through which the suture 10 passes. Fig. 1C is a diagram in which the protective portion 2 inserted into the bone hole H1 is viewed from the front side.

Specifically, the protective portion 2 has a straight groove-like channel 2b that extends in the longitudinal direction of the protective portion 2 over the entire length of the protective portion 2. The channel 2b is formed on a top side of the protective portion 2 and receives the suture 10 from the top side. The pathway P is defined between an inside surface of the channel 2b and an inside surface of the bone hole H1 on the proximal side and is disposed in the bone hole H1 on the proximal side.

The guide portion 3 has a columnar guide hole 3a that specifies the guide axis G and guides the drill that passes through the guide hole 3a along the guide axis G.

Specifically, the guide portion 3 has a guide block 31, which has the guide hole 3a, and a securing knob 32 for securing the guide block 31 to the arm portion 4 and releasing the securing thereof. The guide block 31 has a male screw 3c that protrudes from an outside surface of a block-like body 3b and that passes through a slot 4c (described later) of the arm portion 4 in the top-to-bottom direction. The guide hole 3a passes through the body 3b in a direction orthogonal to the male screw 3c. The securing knob 32 has a female screw 3d to be connected to the male screw 3c.

The inner diameter of the guide hole 3a is substantially equal to the outer diameter of the drill sleeve 13 and the inner diameter of the drill sleeve 13 is substantially equal to the outer diameter of the drill. Therefore, the drill sleeve 13 that passes through the guide hole 3a and the drill that passes through the interior of the drill sleeve 13 are disposed so as to be coaxial or substantially coaxial with the guide axis G, which is a center axis of the guide hole 3a, and the drill that moves forward in the interior of the drill sleeve 13 is guided along the guide axis G. The drill sleeve 13 may be provided as part of the guide device 1 together with the protective portion 2, the guide portion 3, and the arm portion 4;

The arm portion 4 has a linear first portion 41 to which the protective portion 2 is attached in the front-to-rear direction and a second portion 42 having an arc shape or a substantially arc shape, to which the guide portion 3 is attached. The first portion 41 is disposed on the front side of the tibia A and the second portion 42 is disposed over the front side of the tibia A to the inside thereof.

The first portion 41 has a rod shape or a columnar shape that extends in the front-to-rear direction and is provided with, in a distal-end portion (rear-end portion) of the first portion 41, a connecting portion 4a (see Fig. 2A) that is attachable to and detachable from the connecting portion 2a of the protective portion 2. In one configuration example, the connecting portions 2a and 4a are formed from a combination of a depression and a protrusion that are fitted to each other.

In order to prevent the protective portion 2 and the first portion 41 from rotating relative to each other about an axis in the longitudinal direction of the first portion 41, it is preferable that lateral cross sections of the depression 2a and the protrusion 4a have noncircular shapes. In Fig. 2A, the lateral cross sections of the depression 2a and the protrusion 4a have substantially semicircular shapes. The connecting portions 2a and 4a may have other arbitrary shapes so long as it is possible to connect the protective portion 2 and the first portion 41 with each other so that the two components do not rotate relative to each other.

On an outside surface on the top side of the first portion 41, a groove-like channel 4b that extends in the longitudinal direction of the first portion 41 over the entire length of the first portion 41 is provided. In the state in which the protective portion 2 is connected to the distal end (rear end) of the first portion 41 in series by means of the connecting portions 2a and 4a, the channel 4b is continuous with the channel 2b, and the channels 2b and 4b extend in a straight line from the distal end (rear end) of the protective portion 2 to the basal end (front end) of the first portion 41.

The second portion 42 has a strip-like shape that extends in an arc-like manner or a substantially arc-like manner toward the inside and the rear side of the first portion 41, and an outside end of the second portion 42 is secured to the first portion 41. Therefore, the guide portion 3 attached to the second portion 42 is disposed inside with respect to the protective portion 2 attached to the first portion 41. The second portion 42 has an arc-shaped or substantially arc-shaped slot 4c that extends in the extension direction of the second portion 42 and that passes through the second portion 42 in the thickness direction (top-to-bottom direction) thereof.

The male screw 3c that passes through the slot 4c is movable in the slot 4c in the extension direction of the slot 4c and is rotatable about a rotation axis I which is the center axis of the male screw 3c and that extends in the top-to-bottom direction. The securing knob 32 is connected to a distal end of the male screw 3c protruding from the slot 4c.

By tightening the securing knob 32, the guide portion 3 is secured to the second portion 42.

On the other hand, by loosening the securing knob 32, the guide portion 3 is released from the secured state with respect to the second portion 42, and the guide portion 3 is supported by the second portion 42 in a movable manner along the extension direction of the slot 4c and a rotatable manner about the rotation axis I. In the state in which the securing knob 32 is loosened, a user can change the position at which the guide portion 3 is attached to the second portion 42 and the rotational angle of the guide portion 3 about the rotation axis I, and, by doing so, the user can adjust the position and the angle of the guide axis G. Fig. 1A shows the preferable position and angle of the guide axis G. When using the guide device 1, the guide axis G is disposed so as to intersect the protective portion 2 in a plan view viewed in the top-to-bottom direction.

Fig. 3 shows the guide device 1 viewed along the guide axis G from the outside or the rear side. As shown in Figs. 1B and 3, the protective portion 2 and the guide axis G respectively extend in parallel or substantially parallel to a plane F that is defined by the arc-shaped second portion 42 and that is orthogonal to the top-to-bottom direction. The guide axis G is disposed at a twist position with respect to the protective portion 2 and passes through on the bottom side of the protective portion 2. In addition, the distance from the guide axis G to an outside surface on the bottom side of the protective portion 2 in the top-to-bottom direction is greater than the radii of a drill 14 and the screw 12a. Therefore, regardless of the position at which the guide portion 3 is attached to the second portion 42 and the rotational angle at which the guide portion 3 is disposed, the drill 14 that is guided along the guide axis G passes through on the bottom side of the protective portion 2 without interfering with the protective portion 2 and the pathway P. Similarly, the screw 12a that has an outer diameter substantially equal to that of the drill 14 and that is fastened into the bone hole H2 formed by means of the drill 14 also passes through on the bottom side of the protective portion 2 without interfering with the protective portion 2 and the pathway P.

Next, a method for using the guide device 1 will be described with reference to Figs. 4A-4D. Note that, in Figs. 4A-4D, the illustration of the osteotomy portion D is omitted.

As shown in Fig. 4A, the protective portion 2 is inserted into the bone hole H1 through which the suture 10 passes, and the suture 10 is accommodated in the channel 2b disposed on the proximal side of the bone hole H1.

Next, as shown in Fig. 4B, the guide device 1 is assembled by attaching the arm portion 4 attached to the guide portion 3 to the protective portion 2, and the drill sleeve 13 that passes through the guide hole 3a is connected to the screw hole 11a to secure the drill sleeve 13 to the bone plate 11. Next, the position and the angle of the bone plate 11 are adjusted with respect to the tibia A in the state in which the securing knob 32 is loosened, and the securing knob 32 is tightened to secure the guide portion 3 to the arm portion 4.

In addition, the suture 10 passing through the channels 2b and 4b is pulled forward, and the suture 10 is held in a tense state.

Next, as shown in Fig. 4C, the bone hole H2 is drilled at the position of each of the screw holes 11a-11h, the screws 12 are inserted into the screw holes 11b-11h other than the screw hole 11a to secure the bone plate 11 to the tibia A. Specifically, by connecting the drill sleeve 13 to each of the screw holes 11a-11h, a plurality of bone holes H2 are drilled in the tibia A via the interior of the drill sleeve 13 and the screws 12 are screwed into the bone holes H2 via the screw holes 11b-11h. At this time, the drill inserted into the drill sleeve 13 at the screw hole 11a is guided along the guide axis G and drills the bone hole H2 along the guide axis G.

In the case in which the gap between the screw hole 11a and the screw hole 11b adjacent to the screw hole 11a is small, the guide block 31 could interfere with the drill sleeve 13 connected to the screw hole 11b. In order to prevent this interference, the guide block 31 may be configured in an inclined manner with respect to the male screw 3c so that the guide block 31 secured to the second portion 42 is inclined outward.

Next, as shown in Fig. 4D, the arm portion 4 is separated from the protective portion 2, and the arm portion 4 and the guide portion 3 are removed, leaving the protective portion 2 in the bone hole H1 in the tibia A. Then, by using a screwdriver 15, the screw 12 is fastened into the bone hole H2 via the screw hole 11a.

In this case, with this embodiment, the guide axis G is disposed at a twist position with respect to the protective portion 2 and the pathway P in the bone hole H1, and it is ensured that there is a sufficient distance between the guide axis G and the pathway P such that the drill being guided along the guide axis G does not interfere with the protective portion 2 and the pathway P. Therefore, a surgeon can easily determine the position of the bone hole H2 so that the drill does not interfere with the suture 10 in the bone hole H1. Also, simply by moving the drill forward in accordance with the guiding by the guide portion 3 and the drill sleeve 13, the surgeon can easily drill the bone hole H2 in the vicinity of the bone hole H1 through which the suture 10 passes, while preventing the drill from interfering with the suture 10. Furthermore, the surgeon can easily insert the screw 12a, which has an outer diameter substantially equal to that of the drill, into the bone hole H2 without interfering with the suture 10.

Furthermore, because the channel 2b is open on the opposite side from the guide axis G, the protective portion 2 is interposed between the suture 10 disposed in the pathway P and the drill and the screw 12a that are guided along the guide axis G. Therefore, it is possible to even more reliably prevent the drill and the screw 12a from interfering with the suture 10.

In addition, because the shape and the dimensions of the tibia A are different in each patient, the bone plate 11 is disposed at the tibia A in a different manner in each patient, and, accordingly, the position and the inclination of the screw hole 11a are different in each patient. With this embodiment, the guide portion 3 supported by the arm portion 4 is movable between the front side and the inside of the tibia A and is rotatable about the rotation axis I that extends in the longitudinal direction of the tibia A. Therefore, it is possible to easily secure the drill sleeve 13 that passes through the guide hole 3a to the screw hole 11a optimally positioned in accordance with the form of the tibia A of each patient, and thus, it is possible to apply the guide device 1 to tibias A of various forms.

In addition, with this embodiment, the guide portion 3 is disposed on the inside with respect to the protective portion 2 and the guide axis G intersects the protective portion 2 in plan view in the top-to-bottom direction, and it is consequently possible to realize the arrangement of the bone hole H1 and the screws 12a in which the tibia A and the medial meniscus MM both can be stably secured.

Specifically, it is preferable that the bone plate 11 be secured to a rear-side portion of the tibia A by means of the screw 12c in order to firmly and stably secure the tibia A by means of the bone plate 11, because a large load is exerted to the rear-side portion of the tibia A and the tibia A is especially hard in the rear-side portion.

Meanwhile, in the pull-out method, a so-called cheese cut sometimes occurs in the vicinity of an opening of the bone hole H1 on the joint surface B. A cheese cut occurs as a result of the suture 10 or the like digging hard into a bone and could result in loosening of the suture 10 and a reduction of the securing force on the medial meniscus MM. In order to prevent the occurrence of a cheese cut, it is preferable that an angle ***θ*** (see Fig. 5) formed between the bone hole H1 and the medial meniscus MM is not too acute (not too small). For example, in the case in which the bone hole H1 is drilled in the rear-side portion of the tibia A, between screws 12a and 12b, or between screws 12b and 12c, an acute angle 6 is formed and a cheese cut is more likely to occur.

With this embodiment, it is possible to form the bone hole H2 for the screw 12a so that an opening O1 of the bone hole H1 on the front surface of the tibia A is disposed farther forward than the screw 12a on the most front side and so that the bone hole H1 passes through the vicinity of said screw 12a without interfering with the screw 12a. Accordingly, it is possible to form the bone hole H1 on the front surface of the tibia A so that the bone plate 11 is disposed at a position closer to the rear side on the inside surface so that the screw 12c passes through the rear-side portion of the tibia A and so that a gentle angle 6 is formed, and thus, it is possible to achieve both firm and stable securing of the tibia A by means of the bone plate 11 and firm and stable internal securing of the medial meniscus MM by means of the pull-out method.

In this embodiment, as shown in Fig. 1D, the protective portion 2 may have a thick first portion 21 on the basal-end side (front side) and a thin second portion 22 on the distal-end side (rear side).

The first portion 21 is substantially columnar. The second portion 22 is thinner than the first portion 21 in the top-to-bottom direction and a bottom-side surface of the second portion 22 is offset to the opposite side (in other words, top side) from the guide axis G with respect to the bottom-side surface of the first portion 21. Therefore, as shown in Fig. 1E, in a state in which at least a distal-end portion of the first portion 21 and the second portion 22 are inserted into the bone hole H1, the second portion 22 is disposed on the proximal side of the bone hole H1 and it is ensured that there is a space on the distal side of the second portion 22.

With this configuration, it is possible to prevent the drill guided along the guide axis G and the screw 12a from interfering with the suture 10, while the guide axis G is brought close to the channel 2b until reaching the position at which a top-side portion of the bone hole H2 and a bottom-side portion of the bone hole H1 interfere with each other.

In the above-described embodiment, although the protective portion 2 and the guide portion 3 are respectively detachable from the arm portion 4, alternatively, at least one of the protective portion 2 and the guide portion 3 may be formed as a single member with the arm portion 4.

For example, in the case in which the positional relationship between the bone hole H1 and the bone hole H2 is set in advance and there is no need to adjust the position and the rotational angle of the guide portion 3 during a surgery, the guide portion 3 and the arm portion 4 may be formed from a single member. In this case, the slot 4c and the securing knob 32 are not necessary.

The protective portion 2 and the arm portion 4 may be formed from a single member. In this case, by rotating the protective portion 2 about the longitudinal axis of the protective portion 2 in the bone hole H1 when connecting the head portion of the screw 12a to the screw hole 11a, the arm portion 4, the guide portion 3, and the drill sleeve 13 are moved to positions at which said components do not interfere with fastening of the screw 12a. At this time, the channel 2b may be coaxially formed with the longitudinal axis of the protective portion 2 so that the pathway P does not rotationally move in the bone hole H2 in association with the rotation of the protective portion 2.

In the above-described embodiment, the protective portion 2 has the groove-like channel 2b opening on the outside surface of the protective portion 2; however, the protective portion 2 may be in other arbitrary forms so long as the protective portion 2 is capable of defining, in the bone hole H1, the pathway P of the suture 10 passing therethrough in the longitudinal direction.

For example, the protective portion 2 may be a cylindrical member and the channel 2b may be a through-hole that passes through the protective portion 2 in the longitudinal direction. In accordance with the form of the channel 2b, the form of the channel 4b of the first portion 41 is changed, as appropriate.

Alternatively, the protective portion 2 may not have the channel 2b. For example, the protective portion 2 may be a substantially semi-columnar member that occupies about half of the interior of the bone hole H1 and may be a flat plate-like member that partitions the interior of the bone hole H1 into two spaces in the radial direction.

In the above-described embodiment, the drill sleeve 13 is inserted into the guide hole 3a of the guide portion 3; however, the drill sleeve 13 is not necessarily needed.

For example, instead of using the drill sleeve 13, an arbitrary means for securing the guide portion 3 with respect to the bone plate 11 at a position at which the guide axis G is disposed so as to be coaxial with the screw hole 11a may be used. In the case in which only the drill is inserted into the guide hole 3a, the inner diameter of the guide hole 3a is designed in accordance with the outer diameter of the drill.

In the above-described embodiment, the guide device 1 is used in the pull-out method and the HTO; however, the usage of the guide device 1 is not limited thereto. Specifically, the guide device 1 may be used in an arbitrary surgery in which a second bone hole is drilled in the vicinity of a first bone hole through which a suture passes.

### {Reference Signs List}

1 guide device
2 protective portion
21 first portion
22 second portion
2b channel
3 guide portion
3a guide hole
4 arm portion
41 first portion
42 second portion
10 suture
14 drill
A tibia
F plane
G guide axis
H1 first bone hole
H2 second bone hole
I rotation axis
P pathway

## Claims

1. A guide device that guides a drill that drills a second bone hole in a bone in which a first bone hole is formed, the guide device comprising:
an elongated protective portion that is inserted into the first bone hole and that defines, in an interior of the first bone hole, a pathway that extends in a longitudinal direction of the first bone hole and through which a suture passes;
a guide portion that has a guide hole that specifies a guide axis and that guides the drill passing through the guide hole along the guide axis; and
an arm portion that connects the protective portion with the guide portion, wherein
the guide axis is disposed at a twist position with respect to the protective portion, and the drill guided along the guide axis passes through a position at which the drill does not interfere with the protective portion and the pathway.

2. The guide device according to Claim 1, wherein:
in the guide device, there are a top-to-bottom direction, a left-to-right direction, and a front-to-rear direction that substantially correspond to a longitudinal direction, a left-to-right direction, and a front-to-rear direction of a tibia, respectively;
the protective portion extends in the front-to-rear direction;
the guide portion is disposed, with respect to the protective portion, on a side corresponding to an inside of the tibia in the left-to-right direction of the guide device; and,
the guide axis intersects the protective portion in plan view viewed in the top-to-bottom direction.

3. The guide device according to Claim 1 or 2, wherein:
the protective portion has a channel that extends in a longitudinal direction of the protective portion over an entire length of the protective portion and that receives the suture; and
the channel opens on an outside surface of the protective portion on an opposite side from the guide axis.

4. The guide device according to any one of Claims 1 to 3, wherein:
the protective portion, the guide portion, and the arm portion are formed from separate members;
the protective portion is attached to the arm portion in a detachable manner; and
the guide portion is attached to the arm portion in a detachable manner.

5. The guide device according to any one of Claims 1 to 4, wherein:
the arm portion has
a first portion that is disposed on a front side of the tibia and to which the protective portion is attached in a front-to-rear direction of the tibia and
a second portion that extends in an arc-like manner or a substantially arc-like manner inward from the front side of the tibia and to which the guide portion is attached; and
the guide portion is supported by the second portion so as to be movable in an extension direction of the second portion and rotatable about a rotation axis that intersects a plane defined by the second portion.

6. The guide device according to any one of Claims 1 to 5, wherein:
the protective portion has a first portion on a basal-end side and a second portion on a distal-end side; and
the second portion is thinner than the first portion and an outside surface on a side of the guide axis in the second portion is offset in a direction away from the guide axis with respect to an outside surface on a side of the guide axis in the first portion.
